(19) Europäisches Patentamt
European Patent Office
Office européen des brevets

(11) Publication number : 0 436 308 B1

# (12) EUROPEAN PATENT SPECIFICATION

(45) Date of publication of patent specification : 11.05.94 Bulletin 94/19

(51) Int. Cl.$^5$ : A61L 17/00

(21) Application number : 90313150.6

(22) Date of filing : 04.12.90

## (54) Surgical filament.

(30) Priority : 11.12.89 JP 318822/89

(43) Date of publication of application : 10.07.91 Bulletin 91/28

(45) Publication of the grant of the patent : 11.05.94 Bulletin 94/19

(84) Designated Contracting States : DE FR GB IT

(56) References cited : FR-A- 2 297 051

(73) Proprietor : MITSUI TOATSU CHEMICALS, Inc.
2-5 Kasumigaseki 3-chome
Chiyoda-Ku Tokyo 100 (JP)

(72) Inventor : Shinoda, Hosei
3-18-1 Takamori Dai
Kasugai-shi, Aichi-ken 487 (JP)
Inventor : Iimuro, Shigeru
5-6 Takiharu-cho, Minami-ku
Nagoya-shi, Aichi-ken 457 (JP)
Inventor : Ohtaguro, Masami
54 Aza Santakane, Narumi-cho
Midori-ku, Nagoya-shi, Aichi-ken 458 (JP)

(74) Representative : Stuart, Ian Alexander et al
MEWBURN ELLIS York House 23 Kingsway
London WC2B 6HP (GB)



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a surgical filament such as a surgical suture or ligature having improved surface-slipping characteristics, for example, ability to pass through the tissue and tie down property.

Various surgical filaments such as sutures and ligatures are used in surgery for the suture, fixation and ligation of tissues including the internal organs, skin, muscles, bones, joints and blood vessels.

These surgical filaments are sometimes used for sutures and ligatures in the form of a monofilament. However, these filaments are often used in the form of multifilament or as a braided or twisted structure.

Even in the case of a monofilament having relatively good surface-slipping characteristics, untreated surfaces of the monofilament often exhibit insufficient smoothness as such. Consequently, tissue is sometimes damaged in the course of suturing or fixation by non-negligible friction between the filament and tissue. In order to prevent such trouble or to slide a knot of the suture to a desired position, various coatings are applied to the surgical filaments.

For example, Japanese Patent Publication SHO 60-25974(1985) discloses a synthetic multifilament suture covered with a mixture composed of a metal salt of a higher fatty acid and a bioabsorbable polymer. Japanese Laid-Open Patent SHO 61-76163(1986) describes a surgical filament dry-coated with a metal salt of a higher fatty acid.

The surgical filaments are, in use in the course of surgery, usually exposed to body fluids or passed through wet tissue from once to several times before making a knot.

When a conventionally known coating agent, for example, calcium stearate is coated on a bioabsorbable suture, a certain extent of the lubricating effect can be observed in the dry state. On the other hand, the surface-slipping characteristics are sometimes impaired in the wet state and thus it becomes difficult to slide a knot of the suture to the desired position. Additionally, there is a problem that passing the filament through the tissue is unsatisfactory.

### Brief Description of the Drawings

Figure 1 is a drawing illustrating a manner of tying a filament specimen and the tensile direction in the tie down test.

Figure 2 is a drawing illustrating a schematic view of the equipment used for the tie down test. In the drawing, 1 is a section of a bar, 2 is a filament, 2a and 2b are both ends of the filament, 3 is a knot, 4 is a sponge, 5a and 5b are chucks of a tensile tester, 6 is a load cell and 7 is a recorder.

### Summary of the Invention

An object of the present invention is to provide a surgical filament having improved surface-slipping characteristics by use of a specific coating agent. A preferred embodiment may provide a surgical filament having surface-slipping characteristics such as tie down property and passing ability through tissue in the wet state substantially equal to those in the dry state and also having a high safety for the organism.

As a result of carrying out an intensive investigation to accomplish the above object, the present inventors have found that a surgical filament having excellent surface-slipping characteristics such as outstanding tie down property and passing ability through tissue in the wet state by coating the surgical filament with a basic amino acid having a long chain acyl group. Thus the present invention has been completed.

Accordingly, one aspect of the present invention is a surgical filament comprising a coated surface with at least one N-long chain monoacylated basic amino acid which has an aliphatic acyl group of from 6 to 22 carbon atoms or with a composition containing at least one of said N-long chain monoacylated basic amino acid.

The N-long chain monoacylated basic amino acid which has an aliphatic acyl group of from 6 to 22 carbon atoms and is preferably used in the surgical filament is a compound represented by the formula ( I ):

$$R\text{-}CONH(CH_2)_n\underset{\displaystyle NH_2}{\underset{|}{C}}HCOOH \qquad (\mathrm{I})$$

wherein R is a long chain alkyl group having from 5 to 21 carbon atoms and n is an integer of from 1 to 4.

The N-long chain monoacylated basic amino acid used in the invention may be coated singly on the filament or may be used as a component of a coating composition composed of several components. In the composition containing at least one of the N-long chain monoacylated basic amino acid, bioabsorbable polymers

are preferably used as other components. The bioabsorbable polymers include, for example, polyglycolic acid, polylactic acid and a copolymer of glycolic acid and lactic acid which are polymers of glycolide and/or lactide.

The raw material of the filament is preferably composed of bioabsorbable polymers which include, for example, polyglycolic acid, polylactic acid, glycolide-lactide copolymers, and additionally glycolide-caprolactone copolymers, polyhydroxy-butyric acid, poly-p-dioxanone and trimethylene carbonate polymers.

The surgical filament of the invention may be coated with the N-long chain monoacylated basic amino acid having an aliphatic acyl group of from 6 to 22 carbon atoms neat. As an alternative method, at least one of the N-long chain monoacylated basic amino acid having an aliphatic acyl group of from 6 to 22 carbon atoms or an admixture thereof with other bioabsorbable polymers is dissolved or suspended in water or a volatile solvent. The surgical filament is dipped into the liquid thus obtained and coated so as to obtain a coat weight of from 0.1 to 20 parts by weight per 100 parts by weight of the filament. Successively the solvent is removed to obtain a coated filament.

When the term "coating" is used in the invention, coating is not necessarily required to cover the whole surface of the filament. Mottled covering or spotted adhesion of the coating agent may also be partially found on the filament surface.

## Description of the Preferred Embodiments

The N-long chain monoacylated basic amino acid used in the invention has a structure of an amino group in a basic amino acid having a plurality of amino groups is acylated with a long chain carboxylic acid.

In such N-long chain monoacylated basic amino acid, the basic amino acid includes, for example, $\alpha,\beta$-diaminopropionic acid, $\alpha,\gamma$-diaminobutyric acid, ornithine and lysine. Lysine exhibits a particularly preferred coating effect, that is, surface-slipping characteristics.

The long chain acyl group preferably has from 6 to 22 carbon atoms and includes, for example, acyl groups derived from caproic acid, enanthic acid, caprylic acid, pelargonic acid, capric acid, lauric acid, myristic acid, palmitic acid, stearic acid, icosanoic acid, docosanoic acid, oleic acid, behenic acid, isostearic acid, coconut oil fatty acid and beef tallow fatty acid. Preferred acids are saturated fatty acids such as lauric acid, myristic acid, palmitic acid and stearic acid. Lauric acid is particularly preferred.

N-Acylated basic amino acids having an acyl group of less than 6 carbon atoms are too hydrophilic and cannot exhibit good coating effects. An acyl group having more than 22 carbon atoms also cannot provide good effects.

Consequently, the N-long chain monoacylated basic amino acid for use in the invention is preferably represented by the formula ( I ):

$$R-CONH(CH_2)_n\underset{NH_2}{\underset{|}{C}}HCOOH \qquad (I)$$

wherein R is a long chain alkyl group of from 5 to 21 carbon atoms and n is an integer of from 1 to 4. A particularly preferred compound is N-$\varepsilon$-lauroyl-L-lysine which has the formula ( II ):

$$C_{11}H_{23}-CONH(CH_2)_4\underset{NH_2}{\underset{|}{C}}HCOOH \qquad (II)$$

The N-long chain monoacylated basic amino acid is prepared, for example, by the Schotten-Baumann reaction in which long chain carboxylic acid chloride is added dropwise to an aqueous alkali solution of basic amino acid or by a method disclosed in Japanese Patent Publication SHO 51-128610(1976) in which the carboxylic acid salt of the basic amino acid is dehydrated by heating.

However, no particular restriction is imposed on the method of synthetis of the N-long chain monoacylated basic amino acid and the amino acid prepared by other methods can also be used without any troubles.

The N-long chain monoacylated basic amino acid has the form of white flaky crystals having a melting point of from about 200 to about 250°C, having excellent lubricating properties, being insoluble or slightly soluble in water and common organic solvents such as chloroform and benzene, and additionally which do not swell and can be maintained in the form of a stable powder. Consequently, frictional resistance on the filament surface is effectively decreased by coating the N-long chain monoacylated basic amino acid on the filament.

Since the N-long chain monoacylated basic amino acid has good water repellency, the surgical filament

surface coated with said substance sheds water effectively when the filament is exposed to body fluids or passes through wet tissue. As a result, the filament can exhibit slipping characteristics substantially equal to those in the dry state.

Further, the N-long chain monoacylated basic amino acid is extremely safe for organisms as understood from its chemical structure. When the long chain monoacyl group is a fatty acid in particular, neither acute nor subacute toxicity is found and any test results on skin irritation, mucous membrane irritation and mutagenicity are negative. Hence, it is reasonable to use said substance for the coating agent.

The surgical filament of the invention may be bioabsorbable or nonbioabsorbable and also may have a monofilament structure or a ultra fine multifilament structure. A surgical filament having a braided structure of a bioabsorbable multifilament in particular exhibits the advantageous effects of the coating of the present invention.

The material of the bioabsorbable surgical filament includes, for example, conventionally known natural substances such as cat gut, collagen and chitin, and synthetic substances such as glycolide polymers (polyglycolic acid), lactide polymers (polylactic acid), glycolide-lactide copolymers, glycolidecaprolactone copolymers, polyhydroxybutyric acid, poly-p-dioxanone and trimethylene carbonate polymers.

Glycolide polymers, lactide polymers and glycolide-lactide copolymers are preferred among these polymers in view of the effect of the coating on improving the surface-slipping characteristics and filament properties such as high strength and good hydrolyzability.

Coating on the filament may be carried out by heat use of the above N-long chain monoacylated basic amino acid. For example, powder of the N-long chain monoacylated basic amino acid is directly adhered without solvent to the surface of the filament. Alternatively, the powder is suspended in a volatile solvent such as chloroform and xylene. Thereafter the filament passes through the suspension and the solvent is evaporated from the filament surface to complete coating.

The N-long chain monoacylated basic amino acid has good adhesion to the surface of the surgical filament and good coating on the filament surface can be carried out in a single step. Hence, the surface-slipping characteristics of the filament can be sufficiently improved even in a small amount.

The N-long chain monoacylated basic amino acid can be mixed with other materials and the composition thus obtained can also be coated on the filament.

Other materials to be mixed with the N-long chain monoacylated basic amino acid preferably include a polymer which is biocompatible and thermoplastic or soluble in a solvent. In particular, a polymer soluble in a solvent provides good workability in the filament coating operation and uniform coating can be achieved.

When the surgical filament is composed of a bioabsobable polymer, the polymer to be mixed with the N-long chain monoacylated basic amino acid is preferably a bioabsorbable polymer.

The bioabsorbable polymer to be mixed includes, for example, glycolide polymers, lactide polymers, glycolide-lactide copolymers, polycaprolactone, polyoxyalkylene, polytetrafluoroethylene and silicone resins. Particularly preferred polymers are bioabsorbable polymers such as glycolide polymers, lactide polymers and glycolide-lactide copolymers.

When the N-long chain monoacylated basic amino acid is mixed with the above polymer and the resultant composition is coated on the surgical filament, the following techniques can be conducted. For example, the glycolic acid-lactic acid copolymer or polycaprolactone is heat-melted at 100 to 200 °C, successively the N-long chain monoacylated basic amino acid is added, and the surgical filament passes through the molten mixture thus obtained to carry out coating. Then the coated filament is cooled in water or in air.

The preferred proportion of the N-long chain monoacylated basic amino acid in the composition is from about 30 to about 200 parts by weight per 100 parts by weight of the above polymer. When the proportion is outside of the above range, adhesion of the composition to the filament surface becomes unfavorably soft and weak.

In another method, the above polymer is dissolved in a solvent such as chloroform and dioxane at a temperature of from room temperature to about 50 °C and successively the N-long chain monoacylated basic amino acid is added to form a suspension. The surgical filament passes through the suspension to carry out coating. The coating temperature is preferably from room temperature to about 50 °C. The filament is immersed in the suspension for 5 seconds to 1 minute and can be coated satisfactorily.

The suspension is prepared by dissolving from 0.01 to 10 parts by weight of the above polymer in 100 parts by weight of the organic solvent, adding from 0.1 to 20 parts by weight of the N-long chain monoacylated basic amino acid, and maintaining the suspension at a temperature of from room temperature to about 50 °C under stirring. Stirring is continued from the preparation of the dispersion to the completion of coating in order to eliminate variations of concentration of the N-long chain monoacylated basic amino acid. After completion of the coating, the filament is dried in air for 1 to 24 hours in the temperature range of from room temperature to about 120°C to remove the organic solvent. Drying under reduced pressure is more effective.

In the surgical filament coated with the composition consisting of a mixture of the N-long chain monoacylated basic amino acid and the above polymer, the above polymer functions as a binder for the N-long chain monoacylated basic amino acid and simultaneously stiffens the filament itself to a moderate extent. Thus the surgical filament obtained can be handled with ease. When the filament has a braided structure of a multifilament in particular, the coating can improve the disadvantage that the multifilament is too flexible and difficult to handle.

When the filament is coated with the composition composed of the N-long chain monoacylated basic amino acid and the above polymer, the amount of the N-long chain monoacylated basic amino acid in the composition is preferably 20 % by weight (component ratio is 1:4) or more. The amount is more preferably 50 % by weight or more, most preferably 80 % by weight or more. When the amount is less than 20 % by weight, good effect of coating, that is, good surface-slipping effects cannot be expected. Hence, particularly in a wet state, it becomes difficult to carry out a smooth tie-down of a knot.

In coating the filament of the invention, other conventionally known lubricants such as calcium stearate or coating agents can be used in combination with the above N-long chain monoacylated basic amino acid. Also in such case, the amount of the N-long chain monoacylated basic amino acid in the coating composition is preferably 20 % by weight or more.

The amount of the N-long chain monoacylated basic amino acid which is coated directly or as a portion of the composition on the filament of the invention varies depending upon the structure of the filament, for example, the number of filament and density of braiding or twisting. Suitable amounts are in the range of from 0.1 to 20 % by weight. An amount less than 0.1 % by weight does not exhibit the good effects of the coating. On the other hand, an amount exceeding 20 % by weight causes the coating agent to fall off in the form of powder and is also unfavorable because of poor appearance and bad economy. The amount is more preferably in the range of from 0.5 to 10.0 % by weight.

The present invention will hereinafter be illustrated further in detail by way of examples. Physical properties in the examples were measured by the following methods.

Tie down test

(1) Subjective method

As to the surgical filament provided by the invention, evaluation of slipping characteristics, particularly tie down property of a knot, can be carried out with extreme ease in a subjective method.

As illustrated in Figure 1, a filament 2 which was passed under a bar 1 was firmly tied at the upper parts and then the resulting knot 3 was pulled down by drawing both ends 2a and 2b of the filament in the directions of the arrows, respectively. The slipping property could be readily evaluated by the ease of pull down.

Tie down property was classified into five grades according to the following standards for evaluation.

0 : Knot does not slide at all or filament is broken.
1 : Knot moves only slightly. Considerable force is required for moving the knot.
2 : Knot moves with intermittent hang up.
3 : Knot slides down with ease.
4 : Knot slides down very smoothly.

(2) Objective method

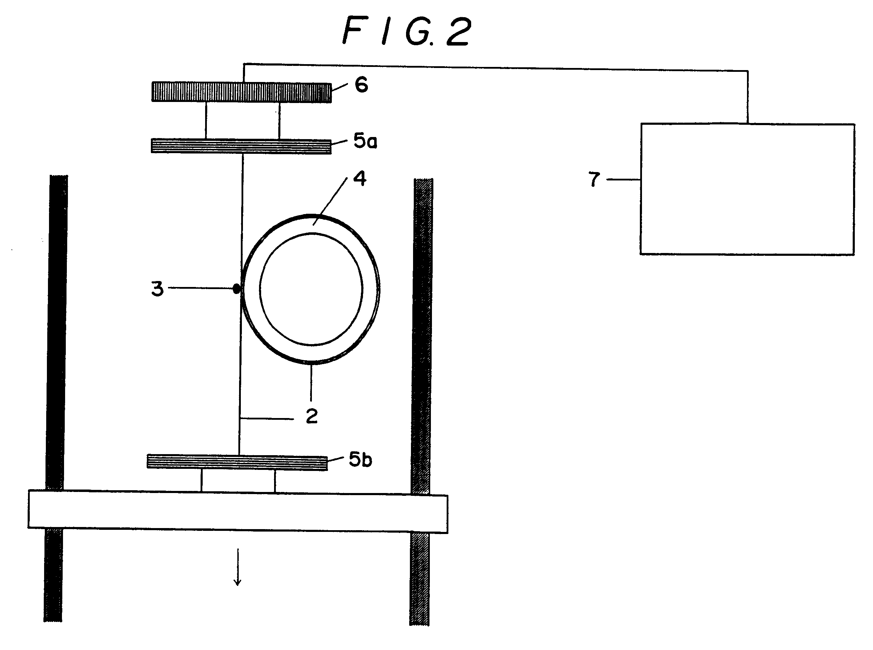

As illustrated in Figure 2, a filament to be tested was turned once around a cylindrical sponge 4 having an inside diameter of 40 mm and an outside diameter of 50 mm and a knot 3 was made as illustrated in Figure 1. Both ends of the filament 2 were respectively fixed to chucks 5a and 5b of a tensile testing machine and drawn at a rate of 100 mm/min. The knot did not slide in some of the filaments 2 and the filament 2 was broken. When the knot could slide, the knot was subjected to slide a distance of 30 cm. The stress detected by a load cell 6 varied depending upon the slide distance of the filament 2. Hence, the initial 10 cm and final 10 cm were omitted from the total slide distance of 30 cm, and maximum and minimum stresses were recorded on the intermediate 10 cm.

Examples 1 - 10 and Comparative Examples 1 - 5

Various coating agents illustrated in Table 1 in powder form were directly adhered to a filament (a kind and size of the filament are shown in Table 1) with a human finger and excess coating agent was wiped off by rubbing the filament with a dry cloth. According to the number of wiping cycles with the cloth, filaments

having various amounts of the coating agents were obtained. The weights of the filaments thus obtained were measured. The adhered amounts of the coating agents were calculated from the difference in weight between the coated and original filaments, and indicated by percentage of the weight of filament.

Tie down tests were carried out on the coated and uncoated filaments as such and slipping characteristics in the dry state were evaluated by the objective method.

Filaments were immersed in distilled water at 37 °C for a minute and tie down tests were carried out to evaluate slipping characteristics by the objective method.

Results are illustrated in Table 1.

Examples 11 - 27 and Comparative Examples 6 - 10

As illustrated in Table 2, solutions of coating agent mixtures were prepared by dissolving 1 g of various resins in the prescribed amount of chloroform and adding N-lauroyl lysine powder in the ratios illustrated in Table 2.

Subsequently, said solutions were stirred to disperse the powder and the same filaments as those used in Example 1 were immersed for about 10 seconds. The filaments were taken out and dried in the air to remove the solvent. Thus coated filaments were obtained.

The weights of the coated filaments were measured, and adhered amount of the coating agents were calculated from the difference of weight between the coated and original filaments. The amount was indicated by wt% of the weight of the filament.

Tie down tests were carried out by the subjective method both in the dry and wet states. Result are illustrated in Table 2.

Example 28

A dispersion was prepared by adding 1 g of N-lauroyl lysine powder to 10 *ml* of xylene and stirring vigorously. The same filament as that used in Example 1 was immersed for 30 seconds in the dispersion thus obtained and then taken out and dried in the air to remove the solvent. Excess coating agent was wiped off with a dry cloth. The calculated amount of adhered coating agent was about 3.9 %.

Slipping characteristics were evaluated on the coated filament by the same procedures as described in Examples 11 - 27. The knot smoothly slide down both in the dry and wet states.

Table 1

| | Filament (size) | Coating agent | (wt %) | Slipping characteristic (kgf) | |
|---|---|---|---|---|---|
| | | | | Dry | Wet |
| Example 1 | PGA (2-0) | N-lauroyl lysine | 7.1 | 0.1 − 0.3 | 0.2 − 0.3 |
| Example 2 | PGA (3-0) | N-lauroyl lysine | 12.1 | 0.1 − 0.2 | 0.1 − 0.2 |
| Example 3 | PGA (2-0) | N-lauroyl lysine | 3.8 | 0.2 − 0.3 | 0.3 − 0.4 |
| Example 4 | PGA (2-0) | N-lauroyl lysine | 0.5 | 0.4 − 0.9 | 0.5 − 1.1 |
| Example 5 | PGA (2-0) | N-lauroyl lysine + Ca-stearate (5/5) | 6.5 | 0.1 − 0.4 | 0.3 − 0.5 |
| Example 6 | PGA (2-0) | N-stearoyl ornithine | 8.3 | 0.2 − 0.4 | 0.2 − 0.4 |
| Example 7 | PGA (2-0) | N-enanthoyl lysine | 9.0 | 0.3 − 0.5 | 0.4 − 0.9 |
| Example 8 | PGLA 9 (2-0) | N-lauroyl lysine | 8.2 | 0.1 − 0.2 | 0.2 − 0.3 |
| Example 9 | PLA (2-0) | N-lauroyl lysine | 6.9 | 0.1 − 0.3 | 0.2 − 0.3 |
| Example 10 | Silk yarn (2-0) | N-lauroyl lysine | 5.6 | 0.2 − 0.4 | 0.2 − 0.5 |
| Comp. Ex. 1 | PGA (2-0) | − | − | ×** | × |
| Comp. Ex. 2 | PGA (2-0) | Ca-stearate | 6.9 | 0.2 − 0.4 | 0.9 − 1.2 |
| Comp. Ex. 3 | PGLA 9 (2-0) | − | − | × | × |
| Comp. Ex. 4 | PLA (2-0) | − | − | × | × |
| Comp. Ex. 5 | Silk yarn (2-0) | − | − | × | × |

Note : * Abbreviations in the filament column are as follows :
PGA : Polyglycolide multifilament suture (braided, dyed)
PLA : Polylactide multifilament suture (braided)
PGLA 9 : Glycolide-lactide copolymer suture (copolymerized ratio = 9/1, braided)
Filament size : In accordance with United States Pharmacopoeia (U.S.P.)
** In the slipping characteristic column:
× : Knot did not slide. Filament was broken.

Table 2

| | Coating agent (g) | | | Chloroform | Coating agent adhered | Tie down evaluation | |
|---|---|---|---|---|---|---|---|
| | N-Lauroyl lysine (A) | Resin component (B)* | (A)/(B) | (mℓ) | (wt %) | Dry | Wet |
| Example 11 | 0.25 | PLA 1.00 | 1/4 | 10 | 29.5 | 3 | 2 |
| 〃 12 | 0.25 | PLA 1.00 | 1/4 | 20 | 9.8 | 3 | 2 |
| 〃 13 | 1.00 | PLA 1.00 | 1/1 | 20 | 15.5 | 4 | 4 |
| 〃 14 | 1.00 | PLA 1.00 | 1/1 | 40 | 3.6 | 3 | 2 |
| 〃 15 | 2.00 | PLA 1.00 | 2/1 | 20 | 18.7 | 4 | 4 |
| 〃 16 | 2.00 | PLA 1.00 | 2/1 | 40 | 5.2 | 3 | 3 |
| 〃 17 | 4.00 | PLA 1.00 | 4/1 | 20 | 23.1 | 4 | 4 |
| 〃 18 | 4.00 | PLA 1.00 | 4/1 | 40 | 9.2 | 4 | 4 |
| 〃 19 | 4.00 | PLA 1.00 | 4/1 | 60 | 3.5 | 4 | 4 |
| 〃 20 | 8.00 | PLA 1.00 | 8/1 | 40 | 14.9 | 4 | 4 |
| 〃 21 | 8.00 | PLA 1.00 | 8/1 | 60 | 4.1 | 4 | 4 |
| 〃 22 | 10.00 | PLA 1.00 | 10/1 | 40 | 16.2 | 4 | 4 |
| 〃 23 | 10.00 | PLA 1.00 | 10/1 | 60 | 4.5 | 4 | 4 |
| 〃 24 | 12.00 | PLA 1.00 | 12/1 | 60 | 4.3 | 4 | 4 |
| 〃 25 | 4.00 | PCL 1.00 | 4/1 | 40 | 8.5 | 4 | 4 |
| 〃 26 | 8.00 | PGLA5 1.00 | 8/1 | 60 | 4.9 | 4 | 4 |
| 〃 27 | 1.00 | - 0 | 1/1 | 20 | 3.6 | 4 | 4 |
| Comp. Ex. 6 | 0.125 | PLA 1.00 | 1/8 | 20 | 8.9 | 2 | 1 |
| 〃 7 | 0.125 | PLA 1.00 | 1/8 | 10 | 33.8 | 0 | 0 |
| 〃 8 | 0 | PLA 1.00 | 0/1 | 10 | 32.5 | 0 | 0 |
| 〃 9 | 0 | PCL 1.00 | 0/1 | 20 | 7.1 | 0 | 0 |
| 〃 10 | 0 | PGLA5 1.00 | 0/1 | 20 | 7.9 | 0 | 0 |

Note : * Abbreviations in resin component column are as follows:

PLA : Polylactide

PCL : Polycaprolactone

PGLA5 : Glycolide-lactide copolymer (copolymerized ratio = 5/5)

## Claims

1. A surgical filament including a surface coated with at least one N-long chain monoacylated basic amino

acid which has an aliphatic acyl group of from 6 to 22 carbon atoms or with a composition containing at least one of said N-long chain monoacylated basic amino acids.

2. The surgical filament of claim 1, wherein the surface is coated with said composition which contains a bioabsorbable polymer.

3. The surgical filament of claim 2, wherein the composition contains at least 20% by weight of said amino acid(s).

4. The surgical filament of claim 2, wherein the composition contains at least 50% by weight of said amino acid(s).

5. The surgical filament of claim 2, wherein the composition contains at least 80% by weight of said amino acid(s).

6. The surgical filament of any preceding claim, wherein the surgical filament is a bioabsorbable polymer.

7. The surgical filament of claim 6, wherein the bioabsorbable polymer is a polymer of at least one of glycolide and lactide.

8. The surgical filament of any preceding claim, where said amino acid is a compound represented by the formula (I):

$$R\text{-}CONH(CH_2)_n\underset{NH_2}{CH}COOH \qquad (I)$$

wherein R is a long chain acyl group having from 5 to 21 carbon atoms, and n is an integer of from 1 to 4.

9. The surgical filament of claim 8, wherein R is an undecyl group and n is 4 in the formula (I).

10. The surgical filament of any preceding claim, wherein the amount of the amino acid coated on the surface is from 0.1 to 20 parts by weight per 100 parts by weight of the filament.

**Patentansprüche**

1. Chirurgischer Faden umfassend eine Oberfläche, die mit zumindest einer langkettig N-monoacylierten basischen Aminosäure, die eine aliphatische Acylgruppe mit 6 bis 22 C-Atomen aufweist, oder mit einer Zusammensetzung beschichtet ist, die zumindest eine der genannten langkettig N-monoacylierten basischen Aminosäuren enthält.

2. Chirurgischer Faden nach Anspruch 1, worin die Oberfläche mit der genannten Zusammensetzung beschichtet ist, die ein bioabsorbierbares Polymer enthält.

3. Chirurgischer Faden nach Anspruch 2, worin die Zusammensetzung zumindest 20 Gew.-% genannter Aminosäure(n) enthält.

4. Chirurgischer Faden nach Anspruch 2, worin die Zusammensetzung zumindest 50 Gew.-% genannter Aminosäure(n) enthält.

5. Chirurgischer Faden nach Anspruch 2, worin die Zusammensetzung zumindest 80 Gew.-% genannter Aminosäure(n) enthält.

6. Chirurgischer Faden nach einem der vorhergehenden Ansprüche, worin der chirurgische Faden ein bioabsorbierbares Polymer ist.

7. Chirurgischer Faden nach Anspruch 6, worin das bioabsorbierbare Polymer ein Polymer von zumindest

einem aus Glykolid und Lactid ausgewählten ist.

8. Chirurgischer Faden nach einem der vorhergehenden Ansprüche, worin genannte Aminosäure eine durch die Formel (I):

$$R-CONH(CH_2)_n\underset{NH_2}{CH}COOH \qquad (I)$$

dargestellte Verbindung ist, worin R eine langkettige Alkylgruppe mit 5 bis 21 C-Atomen und n eine ganze Zahl von 1 bis 4 ist.

9. Chirurgischer Faden nach Anspruch 8, worin in der Formel (I) R eine Undecylgruppe und n 4 ist.

10. Chirurgischer Faden nach einem der vorhergehenden Ansprüche, worin die Menge der auf die Oberfläche aufgetragenen Aminosäure 0,1 bis 20 Gew.-Teile pro 100 Gew.-Teile des Fadens beträgt.

## Revendications

1. Filament chirurgical incluant une surface recouverte avec au moins un acide aminé basique monoacylé à longue chaîne en N qui a un groupe acyle aliphatique de à partir de 6 à 22 atomes de carbone ou avec une composition contenant au moins un desdits acides aminés basiques monoacylés à longue chaîne en N.

2. Filament chirurgical selon la revendication 1, dans lequel la surface est recouverte avec ladite composition qui contient un polymère bioabsorbable.

3. Filament chirurgical selon la revendication 2, dans lequel la composition contient au moins 20% en poids du(des)dit(s) acide(s) aminé(s).

4. Filament chirurgical selon la revendication 2, dans lequel la composition contient au moins 50% en poids du(des)dit(s) acide(s) aminé(s).

5. Filament chirurgical selon la revendication 2, dans lequel la composition contient au moins 80% en poids du(des)dit(s) acide(s) aminé(s).

6. Filament chirurgical selon l'une quelconque des revendications précédentes, dans lequel le filament chirurgical est un polymère bioabsorbable.

7. Filament chirurgical selon la revendication 6, dans lequel le polymère bioabsorbable est un polymère d'au moins un parmi un glycolide et un lactide.

8. Filament chirurgical selon l'une quelconque des revendications précédentes où ledit acide aminé est un composé représenté par la formule (I) :

$$R-CONH(CH_2)_n\underset{NH_2}{CH}COOH \qquad (I)$$

dans laquelle R est un groupe acyle à longue chaîne ayant de 5 à 21 atomes de carbone, et n est un entier de 1 à 4.

9. Filament chirurgical selon la revendication 8, dans lequel R est un groupe undécyle et n est 4 dans la formule (I).

10. Filament chirurgical selon l'une quelconque des revendications précédentes, dans lequel la quantité d'acide aminé recouvert sur la surface est de 0,1 à 20 parties en poids pour 100 parties en poids du filament.

FIG.1

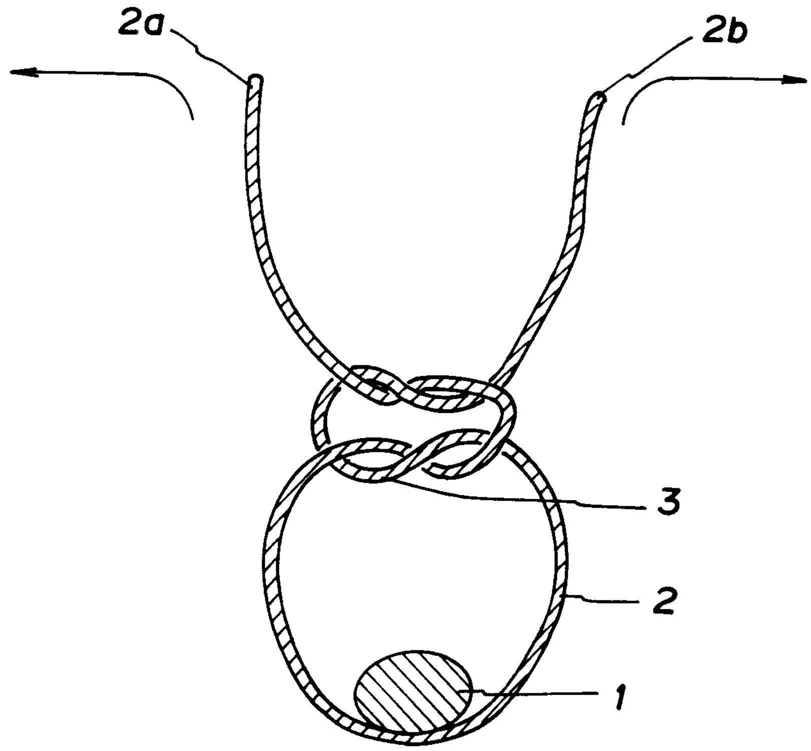

FIG. 2